# EUROPÄISCHE PATENTSCHRIFT

(11) **EP 0 544 275 B1**
(45) Veröffentlichungstag und Bekanntmachung des Hinweises auf die Patenterteilung: **08.07.1998**
(21) Anmeldenummer: 92120129.9
(22) Anmeldetag: 25.11.1992
(51) Int. Cl.: A61B 17/00, A61C 19/00, A61G 15/14

(54) **Ärztliche Behandlungseinrichtung, insbesondere für chirurgische Zwecke**
Medical treatment device, in particular for surgical purposes
Dispositif de traitement médical, en particulier à des fins chirurgicales

(30) Priorität: 25.11.1991 DE 4138673
(43) Veröffentlichungstag der Anmeldung: 02.06.1993
(73) Patentinhaber: KALTENBACH & VOIGT GMBH & CO., 88400 Biberach (DE)
(72) Erfinder: Löhn, Gerd, W-7950 Biberach (DE)
(74) Vertreter: Schmidt-Evers, Jürgen, Dipl.-Ing.

(56) Entgegenhaltungen:
- EP-A- 0 023 009
- GB-A- 2 137 885
- US-A- 3 213 537

## Beschreibung

Die Erfindung bezieht sich auf eine ärztliche Behandlungseinrichtung, insbesondere für chirurgische Zwecke.

Bei der Behandlung des menschlichen oder tierischen Körpers mit einem ärztlichen Behandlungsinstrument ist in vielen Fällen die Zugabe eines gasförmigen oder insbesondere flüssigen Behandlungsmittels erforderlich, wobei es sich hierbei um Luft, Wasser oder ein Gemisch davon (Spray) handeln kann. In vielen Fällen, insbesondere in der chirurgischen und microchirurgischen Behandlung ist eine Kühlflüssigkeit erforderlich, wobei es üblich ist, eine Salzlösung (NaCl) zu verwenden.

Das Behandlungsmittel wird dem Behandlungsinstrument durch eine flexible Rohrleitung, insbesondere eine Schlauchleitung zugeführt. Es ist bereits vorgeschlagen worden, die flexible Rohrleitung am oder in der Versorgungsleitung anzuordnen. Aufgrund ihrer hohlen Ausführung ist die flexible Rohrleitung empfindlich gegen starke Biegungen, was bei der Behandlung oft nicht auszuschließen ist. Es ist deshalb damit zu rechnen, daß die Lebensdauer einer solchen flexiblen Rohrleitung geringer ist, als andere Leitungen, z.B. elektrische Kabel. Wenn man eine defekte flexible Rohrleitung austauscht, dann kann deshalb die Versorgungsleitung selbst weiter verwendet werden. Insbesondere nach einer chirurgischen Behandlung ist eine Sterilisation nicht nur des

Behandlungsinstruments sondern auch der damit fest verbundenen Versorgungsleitung erforderlich, was in vielen Fällen durch Dampfdruck-Sterilisation erfolgt. Insbesondere durch diese thermische Beanspruchung wird die Festigkeit und Lebensdauer der flexiblen Rohrleitung beeinträchtigt.

Insbesondere dann, wenn als Kühlflüssigkeit eine mineralische Lösung, wie z.B. eine Salzlösung, verwendet wird, dann besteht die Gefahr von Kristallisation und Ablagerungen in der flexiblen Rohrleitung, die deren freien Querschnitt verringern oder sogar verstopfen und somit die Kühlung beeinträchtigen können. Diese Gefahr ist umso größer je kleiner der Querschnitt der flexiblen Rohrleitung ist. Eine Demontage der flexiblen Rohrleitung von der Versorgungsleitung ist bei den bekannten Behandlungseinrichtungen mit vertretbarem Aufwand nicht möglich.

In der EP-A-0 023 009 ist ein zahnärztliches Behandlungsinstrument in Form eines Handstücks beschrieben, das aus einem distalen und einem aproximalen Handstückteil besteht, die lösbar miteinander verbunden sind. Das aproximale Handstückteil ist lösbar mit einem sogenannten Versorgungsschlauch verbunden, in dem sich mehrere Kühlmitteilleitungen nach distal und durch das Handstück bis in die Nähe des Werkzeugs erstrecken. Im Bereich des Versorgungsschlauches sind die Leitungen jeweils durch einen flexiblen Schlauch gebildet. Die Anschlüsse der beiden Kühlmittel-Schläuche an das Handstück sind nicht näher beschrieben.

Aus der GB-A-2 137 885 ist ein Behandlungsinstrument ähnlicher Konstruktion beschrieben, wobei das aproximale Handstückteil aus mehreren axial hintereinander angeordneten Abschnitten besteht, wobei nicht beschrieben ist, ob diese Teile lösbar miteinander verbunden sind. Zu erkennen ist, daß das distale Ende des Schlauches in einer Bohrung im aproximalen Handstückteil eingesteckt ist. Ein Durchschieben des Schlauches durch das aproxiale Handstückteil nach distal ist nicht möglich.

In der US-A-3 213 537 ist eine ärztliche Behandlungseinrichtung auch für chirurgische Zwecke mit wenigstens einem Behandlungsinstrument in Form eines Handstücks beschrieben, das einstückig ausgebildet ist. Eine flexible Versorgungsleitung ist an eine aproximale Abschlußkappe des Handstücks angeschlossen, wobei mehrere Schläuche im Bereich einer Abschlußwand mit weiterführenden Leitungen verbunden sind. Auch bei dieser bekannten Ausgestaltung ist ein Herausziehen des oder der Schläuche nach distal nicht möglich.

Der Erfindung liegt die Aufgabe zugrunde, eine ärztliche Behandlungseinrichtung der vorliegenden Art so auszugestalten, daß die flexible Rohrleitung mit geringem Aufwand demontierbar bzw. montierbar oder austauschbar ist.

Diese Aufgabe wird durch die Merkmale des Anspruches 1 gelöst. Vorteilhafte Weiterbildungen der Erfindung sind in den Unteransprüchen beschrieben.

Bei der erfindungsgemäßen Behandlungseinrichtung ist die flexible Rohrleitung nur in ihrem vordere Endbereich im Sinne einer Fassung klemmend und somit dicht an einem vorzugsweise stiftförmigen Fassungsteil befestigt, während sie in ihrem übrigen Bereich mit Bewegungsspiel der Versorgungsleitung aufgenommen ist. Das Fassungsteil selbst ist ebenfalls in einer Steckfassung im Behandlungsinstrument gehalten und insbesondere nach vorne mit der flexiblen Rohrleitung herausziehbar. Die Montage der flexiblen Rohrleitung kann durch Einschieben von vorne erfolgen, was leicht und schnell durchzuführen ist, weil die Steckfassung des Fassungsteils im Querschnitt größer bemessen ist, als die flexible Rohrleitung und deshalb letztere mit Bewegungsspiel aus- und eingeschoben werden kann. Sofern ein Austausch der flexiblen Rohrleitung durchgeführt werden soll, kann vor dem Herausziehen der alten Rohrleitung eine neue Rohrleitung am hinteren Ende der alten Rohrleitung mittels eines zugeordneten Verbindungselements verbunden werden, das ebenfalls mit Bewegungsspiel durch die Versorgungsleitung und die Steckfassung hindurchziehbar ist. Auf diese Weise wird beim Herausziehen der alten Rohrleitung nach vorne die neue Rohrleitung gleichzeitig von hinten nach vorne eingezogen. Es bedarf dann lediglich des Anschlusses des Fassungsteils an das vordere Ende der neuen Rohrleitung und der Positionierung des Fassungsteils im Behandlungsinstrument. Auf diese Weise kann eine Versorgungsleitung mit einer defekten flexiblen Rohrleitung einfach und kostengünstig repariert werden. Es ist auch möglich, die flexible Rohrleitung lediglich zum Zweck ihrer Spülung und/oder Sterilisation in einer von der Versorgungsleitung entfernten Position zu demontieren und dann wieder zu montieren.

Die erfindungsgemäße Lösung eignet sich insbesondere für solche Behandlungsinstruments, die zweiteilig ausgeführt und durch eine Schnellkupplung bzw. Steckvorrichtung voneinander lösbar sind. Bei einer solchen Ausgestaltung befindet sich das die flexible Rohrleitung haltende und fixierende Fassungsteil im hinteren Teil des Behandlungsinstruments, das sich auch dazu eignet, einen Antriebsmotor für ein Behandlungswerkzeug des Behandlungsinstruments aufzunehmen.

In den Unteransprüchen sind Merkmale enthalten, die die vorliegende Problemlösung weiter verbessern und außerdem zu einer einfachen und kostengünstig herstellbaren sowie praktischen und unempfindlichen Bauweise führen, die auch eine gute Abdichtung gewährleistet.

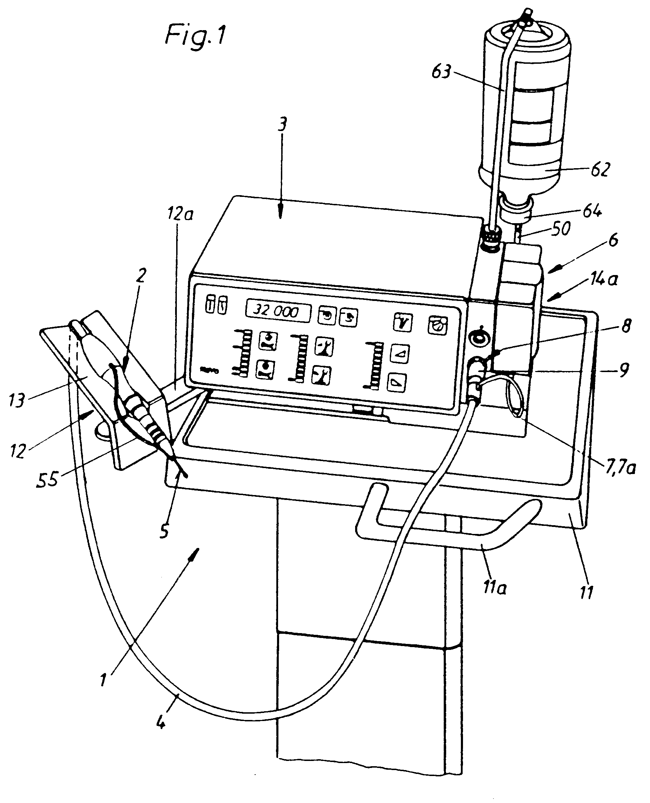

Nachfolgend werden die Erfindung und weitere durch sie erzielbare Vorteile anhand eines bevorzugten Ausführungsbeispiels und einer Zeichnung näher erläutert. Es zeigt
Fig.1 eine erfindungsgemäße Behandlungseinrichtung in perspektivischer Darstellung von vorne;

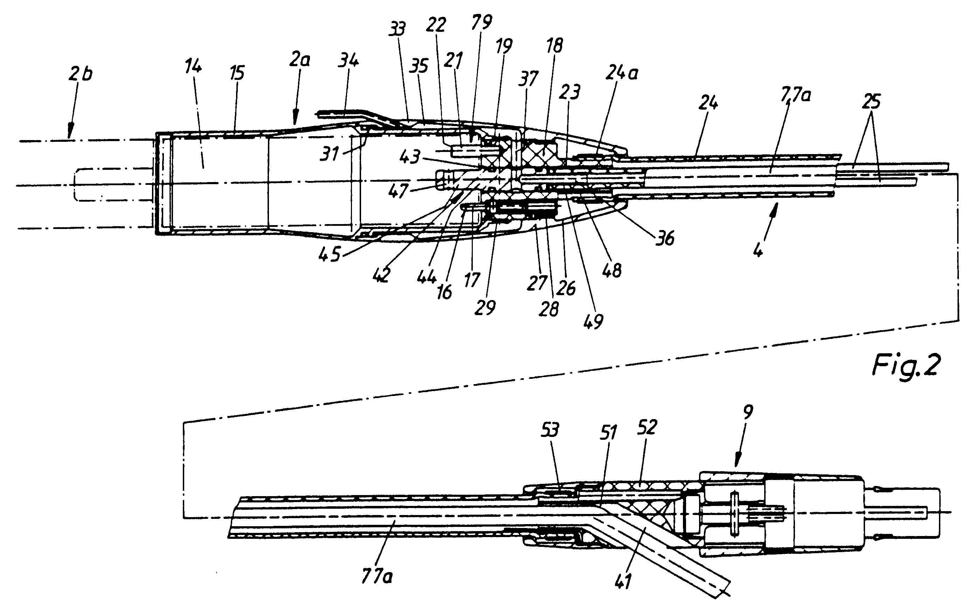

Fig.2 ein Behandlungsinstrument mit zugehöriger Medien-Versorgungsleitung im axialen Schnitt;
Fig. 3 die Anordnung nach Fig. 2 in einer Demontagestellung.

Die Hauptteile der Behandlungseinrichtung 1 sind das Behandlungsinstrument in Form eines Handstücks 2 und ein Steuergerät 3 mit Steuertasten an seiner Frontseite und mit einer integrierten elektronsichen Steuereinrichtung, mit der das Handstück 2 durch die biegsame bzw. flexible Versorgungsleitung 4 verbunden ist. Das Handstück 2 trägt an seinem vorderen Ende ein Behandlungswerkzeug 5, das vorzugsweise gegen wahlweise erforderliche unterschiedliche Werkzeuge austauschbar ist. Bei solchen Behandlungsinstrumenten, bei denen die Zuführung eines Behandlungsmittels, insbesondere einer Kühlflüssigkeit wie eine Salzlösung (NaCl), zur Behandlungsstelle erforderlich ist, ist dem Steuergerät 3 eine Pumpe in Form der Schlauchpumpe 6 zugeordnet, die vorzugsweise seitlich am Steuergerät 3 angebaut oder in dieses integriert sein kann. Die zum vorderen Ende des Handstücks 2 führende Kühlmittelleitung 7 verläuft wenigstens teilweise durch die Versorgungsleitung 4 bzw. das Handstück 2. Am Steuergerät 3 ist eine Steckkupplung 8 angeordnet, in die ein am freien Ende der Versorgungsleitung 4 angeordneter Stecker 9 einsteckbar ist zwecks Anschluß an die elektronische Steuereinrichtung. Zur Positionierung des Steuergeräts 3 kann ein Gestell oder Tisch 11 vorgesehen sein, dem eine Haltevorrichtung 12 für das Handstück 2 in dessen Bereitschaftsstellung zugeordnet ist. Bei der vorliegenden Ausgestaltung ist ein Ablageteil in Form einer muldenförmigen schrägen, zum Behandlungsplatz hin geneigten Platte 13 vorgesehen, auf der das Handstück 2 in einer mit dem Werkzeug 5 schräg nach unten weisenden Position formschlüssig aufliegt. Das Handstück 2 ist aufgrund der muldenförmigen Form der Platte 13 gegen seitliches Verlagern gesichert. Vorzugsweise ist der Muldengrund an die tailliert Form des Handstücks 2 angepaßt, so daß sich auch in dessen Längsrichtung eine formschlüssige Halterung ergibt. Die Platte 13 kann durch einen etwa horizontal, vom Tisch 11 oder auch vom Gehäuse des Steuergeräts 3 wegragenden, vorzugsweise horizontal schwenkbar daran gelagerten und längenveränderlichen Tragarm 12a gehalten sein, an dessen Ende sie vorzugsweise in einem Gelenk um eine etwa vertikale Schwenkachse frei schwenkbar gelagert ist.

Um auch das Ablageteil (Platte 13) einfach sterilisieren zu können, ist es vorteilhaft, das Ablageteil durch eine Schnellkupplung oder Steckfassung zu halten, insbesondere von oben einsteckbar anzuordnen, wozu ein von oben in eine Gelenkbohrung des Tragarms 12a einsteckbarer Gelenkbolzen dienen kann.

Das Handstück 2 besteht aus zwei in seiner Längsrichtung hintereinander angeordneten Teilen nämlich einem an der Versorgungsleitung 4 montierten Antriebsteil 2a und einem an dessen vorderen Ende durch eine Schnellkupplung lösbar befestigbaren Werkzeugträgerteil 2b. Im Antriebsteil 2a ist ein geeigneter Antriebsmotor z.B. ein Elektromotor oder ein druckluftbetriebener Turbinenmotor angeordnet, der in einem Gehäuse in Form einer Motorpatrone 14 kartuschenförmig gekappselt ist. Das Antriebsteil 2a weist zur Aufnahme der Motorpatrone 14 eine vorderseitig offene zylindrische Hülse 15 auf, die so lang bemessen ist, daß sie wenigstens das Gehäuse der Motorpatrone 14 vollständig überdeckt. An der Rückseite der Motorpatrone 14 sind vorzugsweise diametral gegenüberliegend zwei Kontaktelemente, insbesondere in Form von Steckkupplungen 16 vorgesehen, die mit zwei Kontaktelementen in Form von vom Hülsengrund achsparallel vorstehenden Kontaktstiften 17 korrespondieren. Die Kontaktstifte 17 stecken in einem im Querschnitt runden Gehäuse-Einsatzstück 18, das in einem am hinteren Ende der Hülse 15 angeordneten Ringbund 19 sitzt und durch wenigstens eine den nach hinten ragenden Ringbund 19 radial in einem Loch durchfassende Schraube gesichert ist. Das Einsatzstück 18 besteht aus elektrisch nicht leitendem Material, insbesondere Kunststoff. Vom Einsatzstück 18 ragt außerdem ein Positioniertift 21 axparallel vor, der in ein entsprechendes Positionierloch 22 am rückseitigen Ende der Motorpatrone 14 paßt. Der Positionierstift 21 ist bezüglich den Kontakstiften 17 in Umfangsrichtung versetzt angeordnet und zwecks Verdeutlichung in die Schnittebene verlegt, so daß nur ein Kontaktstift 17 sichtbar ist. Das Einsatzstück 18 weist rückseitig einen verjüngten zylindrischen Ansatz 23 auf, auf dem ein biegsamer Schutzschlauch 24 aufgesteckt und durch eine Schlauchklemme 24a oder dergleichen gesichert ist. Im Schutzschlauch 24 erstrecken sich je nach Ausführung mehrere Leiter z.B. zwei elektrische Kabel 25, dessen Adern in nicht dargestellter Weise mit den Kontaktstiften 17 verbunden sind, wenigstens ein Behandlungsmittel - insbesondere Kühlmittellschlauch 7a und ggfs ein Lichtleiter, alle in flexibler Ausführung.

Das Antriebsteil 2a weist zwei zueinander koaxial angeordnete Gehäuseteile auf, nämlich zum einen die Hülse 15 und eine bezüglich dieser rückseitige Hülsenkappe 26, die den hinteren Endbereich oder die hintere Hälfte der Hülse 15 übergreift, das Einsatzstück 18 überdeckt und sich bis in den freien Endbereich des Ansatzes 23 erstreckt, wo sie den Schutzschlauch 24 mit Bewegungsspiel umgibt. Die Hülsenkappe 26 weist in ihrem etwa mittleren Bereich einen Innenringansatz 27 mit einer zylindrischen Innenfläche 28 auf, die vorzugsweise gegenüber der zylindrischen Innenfläche 29 des Ringansatzes 19 im Durchmesser etwas kleiner bemessen ist. Im hinteren Bereich des Einsatzstücks 18 ist zwischen letzterem und dem Ringansatz 27 ein Innengwinde/Außengenwinde vorgesehen, mit dem die Hülsenkappe 26 von hinten aufschraubbar ist. Das vordere Ende der im Querschnitt runden Hülsenkappe 26 sitzt auf einem zylindrischen Außenringansatz 31 der Hülse 15 und ist durch einen in einer Nut vorzugsweise im Außenringansatz 31 angeordneten Dichtungsring (O-Ring) abgedichtet. Auch die Innenflächen des Ringansatzes 19 und Innenringansatzes 27 sind durch jeweils einen O-Ring abgedichtet, der in einer Nut im Einsatzstück 18 sitzt. Hinter dem vorderen freien Randbereich der Hülsenkappe 26 befindet sich ein weiterer Innenringansatz 33, der von einer sich schräg nach vorne und außen erstreckenden Bohrung durchsetzt ist, in dem ein Röhrchen 34 mit seinem hintere Ende fest und dicht eingesetzt ist, das außerhalb der Hülsenkappe 26 etwa achsparallel nach vorne abgewinkelt ist. Zwischen dem Außenringansatz 31 und dem Innenringansatz 27 erstreckt sich zwischen der Hülse 15 und der Hülsenkappe 26 ein freier Ringraum 35, von dem das Röhrchen 34 ausgeht und der einen Abschnitt der Kühlmediumleitung 7 bildet. Letztere ist durch einen axial und einen radial verlaufenden Kanalabschnitt 36, 37 im Einsatzstück 18 mit dem Ringraum 35 verbunden. Zur Abdichtung der Steckfassung sind vor und hinter dem radialen Kanalabschnitt 37 Dichtungselemente vorgeshen, z.B. in Nuten des Paßzylinders 44 angeordnete O-Ringe. Das Röhrchen 34 ist an seinem distalen Ende mit einem Schlauchabschnitt 55 verbunden der durch eine Anschlußarmatur mit dem distalen Endbereich des Handstückteils 2b verbunden ist.

Im Bereich zwischen der Schlauchpumpe 6 und dem Einsatzstück 18 wird die Kühlmittelleitung durch den Kühlmittelschlauch 7a gebildet, der an eine Anschlußarmatur der Schlauchpumpe 6 angeschlossen ist, den Stecker 9 in einem Duchführungsloch 41 radial einwärts durchsetzt und sich dann in der Versorgungsleitung 4 bis zum Einsatzstück 18 erstreckt. Für den Anschluß des Kühlmittelsshlauch 7 an das Einsatzstück 18 ist ein vorzugsweise runder Steckzapfen 42 vorgesehen, der in ein koaxiales, durchgehendes Steckloch 43 im Einsatzstück 18 einsteckbar und darin in axialer Richtung in nicht dargestellter Weise fixierbar ist. Das Steckloch 43 ist bei der vorliegenden Ausgestaltung eine zylindrisch durchgehende Bohrung in der der Steckzapfen 42 mit geringem Bewegungsspiel insbesondere von vorne einsteckbar ist.

Der Steckzapfen 42 besteht aus einem Paßzylinder 44 von dem ein Angriffsglied 45 in Form eines verjüngten Zapfens vorspringt, der in der eingesteckten Position das Einsatzstück 18 nach vorne überragt und ein Angriffselement vorzugsweise in Form einer Umfangsnut 47 aufweist. In der Motorpatrone 14 ist zur Aufnahme des Angriffsgliedes 45 eine Ausnehmung vorgesehen. An der Rückseite des Paßzylinders 44 ist eine hülsenförmige Schlauchtülle 48 angeformt, auf der der Kühlmittelschlauch 7a mit seinem vorderen Ende aufgesteckt und durch eine Schlauchklemme 49 befestigt ist. Der radial verlaufende Kanalabschnitt 37 erstreckt sich teilweise im Einsatzstück 18 und im Paßzylinder 44, wobei der axiale Kanalabschnitt 36 im Steckzapfen 42 koaxial angeordnet ist, rückseitig in die Schlauchtülle 48 ausmündet und den Kühlmittelschlauch 7a mit dem radialen Kanalabschnitt 37 verbindet. Das Steckloch 43, der Schutzschlauch 24 bzw. dessen Freiraum und das Durchführungsloch 41 sind in ihren Querschnittsgrößen so groß bemessen, daß der Kühlmittelschlauch 7a gegebenenfalls mit Schlauchklemme 49 mit Bewegungsspiel hindurchführbar sind. Dabei muß der Kühlmittelschlauch 7a auch ein axiales Loch 51 im hinteren Bereich der Tülle 52 des Steckers 9 durchfassen, auf dem der Schutzschlauch 24 mit einer Schlauchklemme 53 aufgeklemmt ist.

Der Kühlmittelschlauch 7a kann somit in einfacher Weise handhabungsfreundlich und schnell von der Versorgungsleitung 4 demontiert werden. Hierzu ist es lediglich erforderlich, das Antriebsteil 2a zu teilen und die Motorpatrone 14 herauszuziehen. Mit einem von vorne in die Hülse 15 einführbaren zangenförmigen Werkzeug (nicht dargestellt) kann dann das Angriffsglied 45 ergriffen und mit dem Kühlmittelschlauch 7a nach vorne aus der Versorgungsleitung 4 herausgezogen werden. Diese kann dann ggfs. nach weiterer Demontage ihrer Einzelteile ohne den Kühlmittelsschlauch 7a, z.B. in einem Autoklaven bei einer Temperatur von etwa 150°C und einem Druck von beispielsweise 3 bar dampfsterilisiert werden. Die Demontierbarkeit des Kühlmittelsschlauches 7a ermöglicht dies, da letzterer von solchen Temperaturbelastungen feigestellt werden kann, wodurch seine Lebensdauer verlängert wird.

Zur Montage des Kühlmittelschlauchs 7a kann dieser entweder von vorne oder von hinten durch das Durchführungsloch 41 in die Versorgungsleitung 4 eingeführt werden, wobei sein Anschluß an den Steckzapfen 42 vorher oder hinterher erfolgen kann.

Der letzte Arbeitsgang dieser Montage besteht darin den Steckzapfen 42 mit dem daran befestigten Kühlmittelschlauch 7a Von vorne in das Einsatzstück 18 in die richtige Axialposition einzusetzen.

In vergleichbar einfacher Weise ist es auch möglich, den Kühlmittelschlauch 7a auszutauschen. Hierzu wird vorzugsweise ein neuer Schlauch 7b an das rückseitige Ende des Kühlmittelschlauchs 7a mittels eines Kupplungsstücks verbunden, das vorzugsweise durch einen Verbindungsstift 54 gebildet ist, auf den die zugehörigen Enden der Kühlmittelschläuche 7a, 7b aufsteckbar und durch eine gemeinsame oder zwei Schlauchklemmen befestigbar sind. Danach kann der alte Kühlmittelschlauch 7a in vorbeschriebener Weise nach vorne herausgezogen werden, wobei der neue Kühlmittelschlauch 7b gleichzeitig eingezogen wird. Nach Trennung der beiden Schläuche 7a, 7b und Anschluß des neuen Schlauchs 7b an den Steckzapfen 42 kann dieser mit dem neuen Schlauch 7b dann in seine Steckposition im Einsatzstück 18 eingesteckt werden.

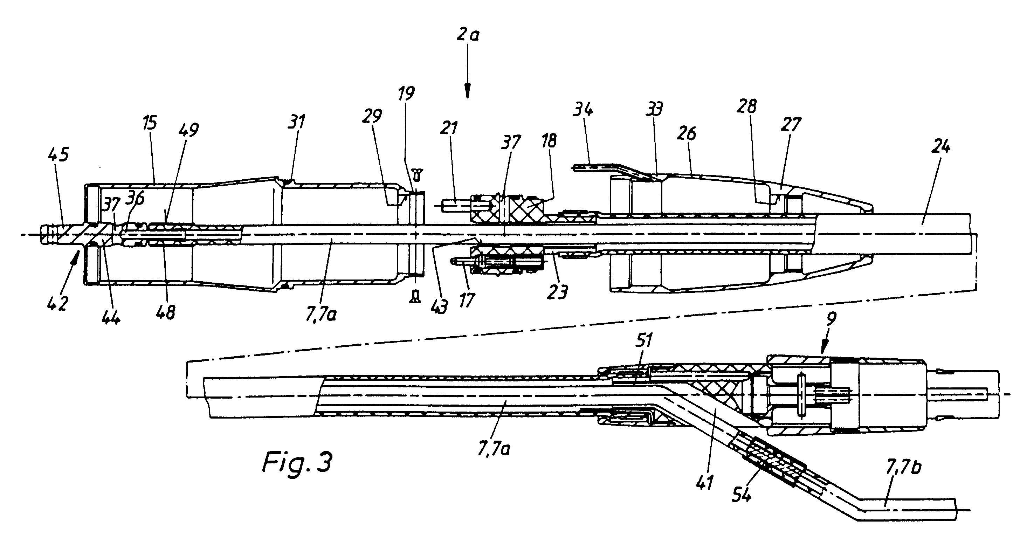

Zwecks weiterer Erleichterung der Montage bzw. Demontage ist es auch möglich, die Hülse 15 und die rückseitige Hülsekappe 26 voneinander zu lösen und so voneinander zu trennen, daß das Einsatzstück 18 mit seinem Angriffsglied 25 frei zugänglich ist, wie es in Fig. 3 dargestellt ist.

## Patentansprüche

1. Ärztliche Behandlungseinrichtung (1), insbesondere für chirurgische Zwecke, mit wenigstens einem Behandlungsinstrument in Form eines Handstücks (2), das durch eine flexible Versorgungsleitung (4) mit einem Steuergerät (3) verbindbar ist, wobei der Behandlungseinrichtung (1) eine Behandlungsmittelquelle (6), insbesondere für Kühlflüssigkeit, zugeordnet ist, an die eine sich wenigstens teilweise durch die Versorgungsleitung (4) zu einem Steckzapfen (42) erstreckende Behandlungsmittelleitung (7) in Form eines Schlauches (7a) anschließbar ist, wobei der Schlauch (7a) sich mit radialem Bewegungsspiel in der Versorgungsleitung (4) bis zum Handstück (2) erstreckt und dort an den Steckzapfen (42) mittels einer Schlauchverbindung (48, 49) angeschlossen ist, wobei der Steckzapfen (42) in einer axialen Steckfassung (18) im Handstück lösbar eingesteckt ist und einen axialen von der Schlauchverbindung (48, 49) ausgehenden Kanalabschnitt (36) aufweist, der an eine zum distalen Ende des Handstücks (2) weiterführende Behandlungmittelleitung (34, 35, 55) angeschlossen ist, und wobei der dem Handstück (2) abgewandte Endbereich des Schlauches (7a) durch ein im dem Handstück (2) abgewandten Endbereich der Versorgungsleitung (4) angeordnetes Durchführungsloch (41) schräg nach hinten oder radial aus der Versorgungsleitung (4) herausgeführt ist.

2. Behandlungseinrichtung nach Anspruch 1,
**dadurch gekennzeichnet,**
daß die weiterführende Behandlungsmittelleitung (34,35,55) als schlauchlos im Handstück (2) verlaufende Leitungsabschnitte ausgeführt sind.

3. Behandlungseinrichtung nach Anspruch 1 oder 2,
dadurch gekennzeichnet,
daß der axiale Kanalabschnitt (36) des Steckzapfens (42) als radialer Kanalabschnitt (37) weitergeführt ist, der an die weiterführende Behandlungsmittelleitung (34,35,55) angeschlossen ist.

4. Behandlungseinrichtung nach einem der vorherigen Ansprüche,
**dadurch gekennzeichnet,**
daß der Steckzapfen (42) in einem Einsatzstück (18) im Handstück (2) angeordnet ist, das in einem axialen Loch (28,29) im Handstück (2) sitzt und wenigstens einen radialen Kanalabschnitt (37) aufweist, der mit einem vom axialen Kanalabschnitt (36) ausgehenden radialen Kanalabschnitt (37) des Steckzapfens (42) korrespondiert.

5. Behandlungseinrichtung nach Anspruch 4,
**dadurch gekennzeichnet,**
daß der Steckzapfen (42) im Einsatzstück (18) vor und hinter den radialen Kanalabschnitten (37) abgedichtet ist.

6. Behandlungseinrichtung nach wenigstens einem der vorherigen Ansprüche,
**dadurch gekennzeichnet,**
daß der Steckzapfen (42) an seinem vorderen Ende ein vorzugsweise durch einen Zapfen gebildetes Angriffsglied (45) aufweist, das im zusammengebanten Zustand nach distal vorragt.

7. Behandlungseinrichtung nach wenigstens einem der vorherigen Ansprüche,
**dadurch gekennzeichnet,**
daß der Steckzapfen (42) an seinem hinteren Ende eine als Schlauchtülle (48) ausgebildete Anschlußarmatur aufweist, durch die er mit dem Schlauch (7a) lösbar verbunden ist.

8. Behandlungseinrichtung nach wenigstens einem der vorherigen Ansprüche,
**dadurch gekennzeichnet,**
daß das Durchführungsloch (41) im Bereich eines rückseitigen Steckers (9) angeordnet ist.

9. Behandlungseinrichtung nach wenigstens einem der vorherigen Ansprüche,
**dadurch gekennzeichnet,**
daß die Versorgungsleitung (4) einen Schutzschlauch (24) aufweist, der mit dem proximalen Ende des Handstücks (2) verbunden ist.

10. Behandlungseinrichtung nach wenigstens einem der vorherigen Ansprüche,
**dadurch gekennzeichnet,**
daß das Handstück (2) mit einem proximalen Handstückteil (2a) und einem distalen Handstückteil (2b) zweistückig ausgeführt ist, wobei die Handstückteile (2a,2b) durch eine Schnellkupplung oder eine Steckfassung miteinander verbindbar sind, und daß die Behandlungsmittelleitung (7) einen die Teilungsfuge zwischen den Handstückteilen (2a,2b) überbrückenden zweiten Schlauchabschnitt (55) aufweist, der durch Anschlußarmaturen mit in den Handstückteilen (2a,2b) verlaufenden Behandlungsmittelleitungsabschnitten verbunden ist.

11. Behandlungseinrichtung nach wenigstens einem der vorherigen Ansprüche,
**dadurch gekennzeichnet,**
daß das proximale Handstückteil (2a) im Bereich des Einsatzstücks (18) mit einem distalen Abschnitt (15) und einem proximalen Abschnitt (26) zweistückig ausgebildet ist, wobei der proximale Abschnitt den distalen Abschnitt auf einem Längsabschnitt übergreift bzw. überlappt und der sich zwischen den Abschnitten (15,26) befindliche Ringhohlraum (35) Teil der Behandlungsmittelleitung (7) ist.

## Claims

1. Medical treatment device (1), particularly for surgical purposes, having at least one treatment instrument in the form of a hand piece (2) which can be connected to a control apparatus (3) by a flexible supply line (4); wherein there is associated with the treatment device (1) a treatment agent source (6), particularly for cooling fluid, to which a treatment agent line (7) in the form of a hose (7a), which line extends at least partially through the supply line (4) to a plug-in spigot (42), can be connected; wherein the hose (7a) extends, with radial clearance of motion, in the supply line (4) as far as the hand piece (2) and is connected, at that point, to the plug-in spigot (42) by means of a hose connection (48, 49); wherein the plug-in spigot (42) is detachably plugged into an axial plug-in socket (18) in the hand piece and has an axial duct portion (36) emanating from the hose connection (48, 49), which duct portion is connected to a treatment agent line (34, 35, 55) continuing to the distal end of the hand piece (2); and wherein that end region of the hose (7a) which is averted from the hand piece (2) is routed obliquely rearwards or radially out of the supply line (4) through a lead-through hole (41) disposed in that end region of the supply line (4) which is averted from the hand piece (2).

2. Treatment device according to claim 1,
**characterised in that**
the continuing treatment agent line (34, 35, 55) is designed as line portions extending in a hose-less manner in the hand piece (2).

3. Treatment device according to claim 1 or 2,
**characterised in that**
the axial duct portion (36) of the plug-in spigot (42) is continued as a radial duct portion (37) which is connected to the continuing treatment agent line (34, 35, 55).

4. Treatment device according to one of the preceding claims, **characterised in that**
the plug-in spigot (42) is disposed in an insert (18) in the hand piece (2), which insert is seated in an axial hole (28, 29) in the hand piece (2) and has at least one radial duct portion (37) which corresponds with a radial duct portion (37) in the plug-in spigot (42), which latter radial duct portion emanates from the axial duct portion (36).

5. Treatment device according to claim 4,
**characterised in that**
the plug-in spigot (42) is sealed, in the insert (18), in front of and behind the radial duct portions (37).

6. Treatment device according to at least one of the preceding claims,
**characterised in that**
the plug-in spigot (42) has, at its front end, an actuating member (45) which is preferably formed by a spigot and, in the assembled condition, projects in the distal direction.

7. Treatment device according to at least one of the preceding claims,
**characterised in that**
the plug-in spigot (42) has, at its rear end, a connecting fitting which is constructed as a hose spout (48) and by which the said plug-in spigot is detachably connected to the hose (7a).

8. Treatment device according to at least one of the preceding claims,
**characterised in that**
the lead-through hole (41) is disposed in the region of a plug (9) at the rear end.

9. Treatment device according to at least one of the preceding claims,
**characterised in that**
the supply line (4) has a protective hose (24) which is connected to the proximal end of the hand piece (2).

10. Treatment device according to at least one of the preceding claims,
**characterised in that**
the hand piece (2) is designed in two pieces with a proximal hand piece part (2a) and a distal hand piece part (2b), the hand piece parts (2a, 2b) being capable of being connected to one another by a high-speed coupling or a plug-in socket, and that the treatment agent line (7) has a second hose portion (55) which bridges the dividing joint between the hand piece parts (2a, 2b) and is connected, by connecting fittings, to treatment agent line portions extending in the hand piece parts (2a, 2b).

11. Treatment device according to at least one of the preceding claims,
**characterised in that**
the proximal hand piece part (2a) is constructed, in the region of the insert (18), in two pieces with a distal portion (15) and a proximal portion (26), the proximal portion engaging over, or overlapping, the distal portion over a longitudinal portion, and the annular cavity (35) located between the portions (15, 26) being part of the treatment agent line (7).

## Revendications

1. Dispositif de traitement (1) médical, en particulier à des fins chirurgicales, comprenant au moins un instrument de traitement sous la forme d'une pièce à main (2) qui peut être relié par une conduite d'alimentation (4) flexible à un appareil de commande (3), dispositif de traitement auquel est associée une source de fluide de traitement (6), en particulier pour un liquide de refroidissement, à laquelle peut être raccordée une conduite de fluide de traitement (7) sous la forme d'un tuyau flexible (7a) s'étendant au moins partiellement à travers la conduite d'alimentation (4) vers un embout à enfichage (42), le tuyau flexible (7a) s'étendant avec du jeu radial de mouvement dans la conduite d'alimentation (4) jusqu'à la pièce à main (2) et étant ici raccordé à l'embout à enfichage (42) à l'aide d'une connexion de tuyau flexible, l'embout à enfichage (42) étant enfiché de façon amovible dans une douille d'enfichage axial (18) dans la pièce à main et présentant un tronçon de canal (36) axiale qui part de la connexion de tuyau flexible (48, 49) et qui est relié à une conduite de fluide de traitement (34, 35, 55) prolongée jusqu'à l'extrémité distale de la pièce à main (2), la partie d'extrémité du tuyau flexible (7), éloignée de la pièce à main (2), sortant de la conduite d'alimentation (4) de façon oblique vers l'arrière ou radialement à travers un trou de passage (41) ménagé dans la zone d'extrémité de la conduite d'alimentation (4) éloignée de la pièce à main (2).

2. Dispositif de traitement suivant la revendication 1, caractérisé par le fait que la conduite de fluide de traitement (34, 35, 55) prolongée est réalisée sous forme de tronçons de conduite s'étendant sans tuyau flexible dans la pièce à main (2).

3. Dispositif de traitement suivant revendication 1 ou 2, caractérisé par le fait que le tronçon de canal axial (36) de l'embout à enfichage (42) est prolongé sous forme de tronçon de canal radial (37) qui est raccordé à la conduite de fluide de traitement (34, 35, 55) prolongée.

4. Dispositif de traitement suivant l'une des revendications précédente, caractérisé par le fait que l'embout à enfichage (42) est disposé dans un insert (18) dans la pièce à main (2), lequel insert est monté dans un trou axial (28, 29) dans la pièce à main (2) et présente au moins un tronçon de canal radial (37) qui correspond avec un tronçon de canal radial (37) de l'embout à enfichage (42), partant du tronçon de canal axial (36).

5. Dispositif de traitement suivant la revendication 4, caractérisé par le fait que l'embout à enfichage (42) est rendu étanche dans l'insert (18) en avant et en arrière des tronçons de canal radial (37).

6. Dispositif de traitement suivant au moins l'une des revendications précédentes, caractérisé par le fait que l'embout à enfichage (42) présente à son extrémité avant un organe de connexion (45) constitué de préférence par un embout et faisant saillie vers le côté distal à l'état assemblé.

7. Dispositif de traitement suivant au moins l'une des revendications précédentes, caractérisé par le fait que l'embout à enfichage (42) présente à son extrémité arrière un raccord réalisé sous forme d'embout (48) par lequel il est relié de façon amovible au tuyau flexible (7a).

8. Dispositif de traitement suivant au moins l'une des revendications précédentes, caractérisé par le fait que le trou de passage (41) est disposé dans la zone d'une fiche arrière (9).

9. Dispositif de traitement suivant au moins l'une des revendications précédentes, caractérisé par le fait que la conduite d'alimentation (4) est un tuyau flexible de protection (24) qui est relié à l'extrémité proximale de la pièce à main (2).

10. Dispositif de traitement suivant au moins l'une des revendications précédentes, caractérisé par le fait que la pièce à main (2) est réalisée en deux parties avec une partie proximale de pièce à main (2a) et une partie distale de pièce à main (2b), les parties de pièces à main (2a, 2b) pouvant être reliée l'une à l'autre par un accouplement rapide ou une connexion à enfichage, et que la conduite de fluide de traitement (7) présente un deuxième tronçon de tuyau flexible (55) qui shunte le joint entre les parties de pièce à main (2a, 2b) et est relié par des raccords aux tronçons de conduite de fluide de traitement s'étendant dans les parties de pièce à main (2a, 2b).

11. Dispositif de traitement suivant au moins l'une des revendications précédentes, caractérisé par le fait que la partie proximale de pièce à main (2a) est réalisée, dans la zone de l'insert (18), en deux parties avec un tronçon distal (15) et un tronçon proximal (26), le tronçon proximal recouvrant ou chevauchant le tronçon distal sur un tronçon de longueur et la cavité annulaire (35) se trouvant entre les tronçons (15, 26) faisant partie de la conduite de fluide de traitement (7).
